# EUROPÄISCHE PATENTSCHRIFT

(11) **EP 0 743 296 B1**
(45) Veröffentlichungstag und Bekanntmachung des Hinweises auf die Patenterteilung: **14.10.1998**
(21) Anmeldenummer: 96107066.1
(22) Anmeldetag: 06.05.1996
(51) Int. Cl.: C07C 35/12, B01J 23/76, B01J 23/78

(54) **Verfahren zur Herstellung von d,I-Menthol**
Process for the preparation of d,I-menthol
Procédé pour la préparation de d,I-Menthol

(30) Priorität: 17.05.1995 DE 19518024
(43) Veröffentlichungstag der Anmeldung: 20.11.1996
(73) Patentinhaber: BAYER AG, 51368 Leverkusen (DE)
(72) Erfinder: Darsow, Gerhard, Dr., 47804 Krefeld (DE); Petruck, Gerd-Michael, Dr., 40699 Erkrath (DE)

(56) Entgegenhaltungen:
- EP-A- 0 563 611
- DE-A- 2 314 813
- CHEMICAL ABSTRACTS, vol. 112, no. 1, 1.Januar 1990 Columbus, Ohio, US; abstract no. 7725, ZUBAREVA ET AL.: "Stereoselective hydrogenation of a menthol-isomenthol mixture on heterogeneous nickel, nickel-cobalt, and cobalt catalysts." XP002012681 & IZV. AKAD. NAUK. SSSR, SER. KHIM., Bd. 8, 1989, Seiten 1920-1923,

## Beschreibung

Die Erfindung betrifft ein Verfahren zur Herstellung von d,l-Menthol aus Verbindungen, die das Kohlenstoffgerüst des Menthans mit wenigstens einer C=C-Doppelbindung besitzen und in 3-Stellung durch Sauerstoff substituiert sind, und/oder aus Stereoisomeren des Menthols in Gegenwart von Wasserstoff und Katalysatoren.

Unter den natürlich vorkommenden cyclischen Terpenalkoholen nimmt l-Menthol aufgrund seiner kühlenden und erfrischenden Wirkung eine Sonderstellung ein. l-Menthol ist der Hauptbestandteil des Pfefferminzöls und wird in der Riechstoff-, Geschmackstoff- und Arzneimittelindustrie eingesetzt.

Die Mentholherstellung durch katalytische Hydrierung von Verbindungen, die das Kohlenstoffgerüst des Menthans mit wenigstens einer C=C-Doppelbindung besitzen und in 3-Stellung durch Sauerstoff substituiert sind (wie z.B. Thymol), führt zum d,l-Racemat, das sich in optische Antipoden spalten läßt. Die 8 optisch aktiven Menthole unterscheiden sich in Bezug auf ihre organoleptischen Eigenschaffen. l-Menthol hat einen charakteristischen Pfefferminzgeruch und die schon erwähnte erfrischende Wirkung; es ist deshalb das wertvollste der Menthol-Stereoisomeren. Man ist daher bestrebt, die Hydrierung so zu führen, daß möglichst viel d,l-Menthol (aus dem l-Menthol durch Racematspaltung gewonnen werden kann) entsteht, oder Stereoisomere des Menthols, wie sie beispielsweise bei der Thymol-Hydrierung anfallen, möglichst effektiv umzulagern.

Aus der DE-AS 2 314 813 und der EP-A 563 611 ist bekannt, daß man aromatische oder teilhydrierte cyclische Verbindungen, die das Kohlenstoffgerüst des Menthans mit wenigstens einer C=C-Doppelbindung besitzen und in 3-Stellung durch Sauerstoff substituiert sind, mit Wasserstoff hydrieren und/oder Stereoisomere des Menthols in Gegenwart von Wasserstoff umlagern kann, wobei ein Cobalt/Mangan-Katalysator bzw. ein Festbettkatalysator, der auf einem mit einem Metall der Seltenen Erden und mit Mangan dotierten Träger Palladium, Ruthenium, Rhodium oder ein Gemisch dieser Elemente als Aktivbestandteile und Alkalimetallhydroxide und/oder -sulfate als Promotoren enthält, eingesetzt wird.

Die US-PS 2 843 636 beschreibt die Isomerisierung von Stereoisomeren des Menthols zu d,l-Menthol mit Wasserstoff in Gegenwart eines Kupferchromitkatalysators.

Diese Verfahren des Standes der Technik erzeugen entweder zu viele Nebenprodukte (die insbesondere bei einer kontinuierlichen Verfahrensweise durch Anreicherung störend ins Gewicht fallen), oder die verwendeten Katalysatoren verlieren zu rasch ihre Anfangsaktivität, sind von eingeschränkter mechanischer Stabilität, sind nur begrenzt belastbar und/oder erschweren eine Wiederaufarbeitung der gebrauchten Katalysatoren.

Es bestand daher der Wunsch, hoch belastbare und langlebige Katalysatoren für die Herstellung von d-Menthol zu d,l-Menthol bereitzustellen, die frei von komplizierten Trägersystemen und daher wieder aufarbeitbar sein sollten.

Überraschenderweise läßt sich das geschilderte Problem mit Hilfe trägerfreier Festbettkatalysatoren lösen, die durch Reduktion von Formkörpern aus verpreßten Metall(hydr)oxidpulvern erhalten werden können. Der Ausdruck "Metall(hydr)oxide" im Sinne dieser Erfindung bedeutet Metalloxide und/oder Metallhydroxide.

Gegenstand der Erfindung ist also ein kontinuierliches Verfahren zur Herstellung von d,l-Menthol durch katalytische Hydrierung von Verbindungen, die das Kohlenstoffgerüst des Menthans mit wenigstens einer C=C-Doppelbindung besitzen und in 3-Stellung durch Sauerstoff substituiert sind, mit Wasserstoff und/oder durch katalytische Umlagerung von Stereoisomeren des Menthols in Gegenwart von Wasserstoff, dadurch gekennzeichnet, daß als Katalysatoren dienende trägerfreie Formkörper verwendet werden, die durch Reduktion von Formkörpern aus verpreßten Pulvern von Cobalt-, Mangan- und Erdalkalimetall(hydr)oxiden und gegebenenfalls einem oder mehreren (Hydr) Oxiden von Metallen der V. und/oder VI. Nebengruppe des Periodensystems der Elemente erhältlich sind.

Die für das erfindungsgemäße Verfahren Verwendung findenden Ausgangsverbindungen sind bekannt (Ullmanns Encyclopädie der technischen Chemie, 3. Auflage, München, 1966, 17. Bd., S. 24, 25; US-PS 2 843 636). Beispielsweise seien genannt: Thymol, Menthon, Menthenon, d- und l-Menthol, d- und l-Neomenthol, d- und l-Isomenthol, d,l-Neomenthol, d,l-Isomenthol. Diese Verbindungen können sowohl einzeln als auch in beliebigen Gemischen verwendet werden.

Für die erfindungsgemäß zu verwendenden Katalysatoren betragen (jeweils berechnet als Metall) die Co-Anteile 40 bis 65 Gew.-%, die Mn-Anteile 10 bis 20 Gew.-%, die Erdalkalimetall-Anteile 0,2 bis 5 Gew.-% und gegebenenfalls die Anteile an Metallen der V. und/oder VI. Nebengruppe des Periodensystems der Elemente (Mendelejew) insgesamt bis zu 7 Gew.-%, vorzugsweise 0,5 bis 7 Gew.-%, insbesondere 1,0 bis 3,5 Gew.-% des gesamten (Hydr)Oxidpulvers. Der Rest zu 100 Gew.-% ist Sauerstoff für die in oxidischer Form vorliegenden Verbindungen. Ein solcher Katalysator kann in dieser Form zwar ohne einen Gehalt von (Hydr)Oxiden der V. und VI. Nebengruppe des Periodensystems für den erfindungsgemäßen Hydrierschritt eingesetzt werden, vorzugsweise erhält er jedoch zusätzlich einen Gehalt von mindestens einem (Hydr)Oxid von Metallen der V. und/oder der VI. Nebengruppe des Periodensystems. Für den Fall des Einsatzes mehrerer (Hydr)Oxide von Elementen der V. und/oder der VI. Nebengruppe des Periodensystems liegt jedes dieser (Hydr)Oxide in einer Menge vor, die nicht kleiner als 20 % und nicht größer als 80 % des genannten Gesamtbereichs von 0,5 bis 7 Gew.-% ist.

Von den Erdalkalielementen eignen sich vor allem Magnesium, Calcium, Strontium und Barium, vorzugsweise Strontium und Barium. Von den Elementen der V. Nebengruppe eignen sich vorzugsweise Vanadium, Niob und Tantal, von den Elementen der VI. Nebengruppe vorzugsweise Chrom, Molybdän und Wolfram. Die als Promotoren wirkenden Elemente der V. und VI. Nebengruppe können entweder einzeln oder als Mischung mehrerer dieser Elemente zum Einsatz kommen.

Die Herstellung der trägerfreien Formkörper kann nach gebräuchlichen Methoden durch Verpressen der Metall(hydr)oxid-Pulvermischungen (gegebenenfalls nach vorausgegangener Temperierung bei höheren Temperaturen), beispielsweise auf Tablettier- oder Pelletier-Maschinen, unter hohem Druck erfolgen, wobei zur Verbesserung des Haftvermögens der Metall(hydr)oxidpartikel auch Graphit und/oder Klebstoffe in Mengen von 0,5 bis 3 Gew.-%, bezogen auf das Gesamtgewicht der zu verpressenden Bestandteile, zum Einsatz kommen können. Beispiele für Formkörper sind Tabletten, Kugeln oder Granulate mit Durchmessern von 3 bis 7 mm. Tablettierte Formkörper können weiterhin zur Vergrößerung der äußeren Oberfläche mit einer axialen Durchbohrung versehen sein. Solche Formkörper haben makroskopisch betrachtet eine glatte Oberfläche.

Die verpreßten Metall(hydr)oxid-Formkörper haben eine hohe Druckfestigkeit von 300 bis 800 N, bevorzugt 400 bis 600 N/cm², auf die plane Formkörperoberfläche bzw. von 50 bis 200 N, bevorzugt 80 bis 140 N, auf die gewölbte Formkörperoberfläche. Die innere Oberfläche der verpreßten Metall(hydr)oxidpulver beträgt 30 bis 200 m²/g, bevorzugt 80 bis 160 m²/g. Die Druckfestigkeit der trägerfreien Formkörper kann nach DIN 50 106, die innere Oberfläche nach F.M. Nelsen und F.T. Eggertsen, Analyt. Chem. 30 (1958), 1387-1392 bzw. S.J. Gregg und S.W. Sing, Adsorption, Surface Area and Porosity, London 1982, Kap. 2 und 6 bestimmt werden.

Vor ihrem Einsatz müssen die Formkörper aus verpreßten (Hydr)Oxidpulvern sorgfältig reduziert werden. Dies geschieht vorzugsweise durch den Einsatz eines Reduktionsgases, das aus einer Inertgas/Wasserstoff-Mischung besteht, in der der Wasserstoffgehalt zu Beginn bei 10 bis 15 Vol.-% liegt. Als Inertgas wird bevorzugt Stickstoff eingesetzt. Die Reduktion erfolgt beispielsweise innerhalb eines Zeitraums von etwa 24 Stunden bei einer Reduktionstemperatur von 180 bis 220°C, wobei in der Endphase der Reduktion der Stickstoffanteil der Gasmischung mehr und mehr vermindert wird, bis diese schließlich aus einem Wasserstoff besteht. Die Reduktion ist beendet, wenn kein Wasserstoff mehr verbraucht und infolgedessen kein Reaktionswasser mehr gebildet wird.

Die Reaktoren können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden (etwa Drahtkörbe oder ähnliches) nützlich sein kann; man kann jedoch auch Hochdruckrohrbündel innerhalb eines gemeinsamen Mantels anwenden, wobei die Einzelrohre wiederum mit den trägerfreien Formkörpern ganz oder teilweise gefüllt werden.

Das erfindungsgemäße Verfahren kann mit den im Festbett angeordneten Katalysatoren in der Gas- bzw. Rieselphase durchgeführt werden, wobei während des Verfahrensablaufs mindestens die 10-fache molare Menge Wasserstoff pro Mol Ausgangsmaterial den Reaktor passiert. Es wird bei 150 bis 230°C, bevorzugt bei 160 bis 210°C und bei einem Druck von 25 bis 350 bar, bevorzugt 100 bis 300 bar, gearbeitet.

Das erfindungsgemäße Verfahren kann mit oder ohne Lösungsmittel durchgeführt werden. Geeignete, unter den Reaktionsbedingungen inerte Lösungsmittel sind beispielsweise Methanol, Ethanol, Isopropanol.

Die stündliche Katalysatorbelastung kann zwischen 400 und 1000 g Reaktionsgemisch pro Liter Katalysator liegen.

Im Rahmen des erfindungsgemäßen Verfahrens sind sehr hohe Katalysatorstandzeiten von 20.000 bis 25.000 Stunden zu erreichen. Diese Standzeiten sind um ein vielfaches höher, als in früheren Veröffentlichungen (z.B. DE-AS 2 314 813) beschrieben.

Die beim erfindungsgemäßen Verfahren erfolgenden Hydrierungen, Racemisierungen sowie Isomerisierungen führen überraschenderweise kaum zur Bildung unverwertbarer Nebenprodukte, wie unerwünschter Kohlenwasserstoffe.

Das erhaltene Reaktionsgemisch enthält einen so hohen Gehalt an d,l-Menthol, daß es durch einfache Destillation auf dieses gewünschte Produkt aufgearbeitet werden kann. Man erhält mit Hilfe des erfindungsgemäßen Verfahrens nicht nur ausgezeichnete Ergebnisse bei der Hydrierung von Thymol, sondern auch ausgezeichnete Ausbeuten bei der Umsetzung der anderen, weiter oben genannten Ausgangsverbindungen.

Nach der destillativen Abtrennung des gewünschten d,l-Menthols kann der Destillationsvorlauf mit dem Destillationssumpf unter Zusatz von frischem Ausgangsprodukt, beispielsweise unter Zusatz von 10 bis 80 Gew.-% Thymol, bezogen auf diesen Destillationsrückstand, wieder in die Reaktion zurückgeführt werden. Die dem destillativ entnommenen d,l-Menthol entsprechende Menge an Ausgangsmaterial wird ersetzt. Der im erfindungsgemäßen Verfahren nicht verbrauchte Wasserstoff kann im Kreis geführt werden.

Das erzeugte d,l-Menthol wird nach der Entfernung des Destillationsvorlaufes und des Destillationssumpfes in einer Reinheit von ≥ 99,9 Gew.-% erhalten und ist deshalb ohne weitere Reinigung für alle weiterverarbeitenden Prozesse einsetzbar.

Das nach der Destillation erhaltene farblose und glasklare Produkt hat einen Schmelzpunkt von 41°C und kann in Kristallisationsgeräten üblicher Bauart zur Kristallisation gebracht werden.

In den nachfolgenden Beispielen bedeutet die Bezeichnung "Nm³": Kubikmeter nach Umrechnung auf Normalbedingungen (1 bar, 25°C).

### Beispiele

### Beispiel 1

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstoff-frei gespült worden war, wurde mit 1,4 l durch Tablettierung von Pulvern von Cobalt-, Mangan-, Barium- und Vanadium(hydr)oxiden hergestellter Formkörper gefüllt. Der Cobaltgehalt der Tabletten lag bei 53 Gew.-%, der Mangangehalt bei 14 Gew.-%, der Bariumgehalt bei 1,1 Gew.-% und der Vanadiumgehalt bei 1,2 Gew.-%. Die Tabletten hatten bei einer Zylinderhöhe von 5 mm und einem Durchmesser von 5 mm eine Druckfestigkeit von 420 N/cm² auf die plane Zylinderoberfläche und von 125 N auf die gewölbte Formkörperfläche sowie eine innere Oberfläche von 168 m²/g.

Die Tabletten wurden zunächst 6 Stunden im Stickstoffstrom getrocknet (Temperatur: max. 200°C, Menge: 5 Nm³ N₂/h). Die Aktivierung erfolgte unter einem Stickstoffdruck von 200 bar bei einer Temperatur zwischen 180 und 220°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil der Gasmischung mehr und mehr vermindert, bis schließlich reiner Wasserstoff den Reaktor durchströmte. Die Aktivierung war beendet, sobald sich kein Reaktionswasser im nachgeschalteten Abscheider mehr ansammelte.

Nach der Aktivierung des Katalysators wurde der Wasserstoffdruck im Reaktorsystem auf 300 bar erhöht. Anschließend wurden stündlich 700 g Thymol (Reinheit: 99,9 Gew.-%) gemeinsam mit 15 Nm³ Wasserstoff unter einem Druck von 300 bar von oben nach unten durch das Hochdruckrohr gepumpt, wobei das Thymol vor Eintritt in das Hochdruckrohr in einem vorgeschalteten elektrisch beheizten Wärmeaustauscher auf eine Temperatur von 170°C erhitzt wurde.

Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Reaktionssystem zurückgeführt wurde, getrennt.

Der Thymol-Durchsatz entsprach einer Katalysatorbelastung von 0,5 kg/l Katalysator x h. Der Katalysator war auch nach einer Laufzeit von 7400 h noch hochwirksam.

Im Hydrierprodukt wurde kein Thymol gefunden.

Nach der destillativen Entfernung der Vor- und Nachsieder wurde das erzeugte d,l-Menthol in einer Reinheit von 99,9 Gew.-% erhalten.

### Beispiel 2

Ein Hochdruckrohr wie in Beispiel 1 wurde unter Inertgas mit 1,4 l durch Tablettierung von Pulvern von Cobalt-, Mangan-, Barium-, Vanadium- und Wolfram(hydr)oxiden hergestellter Formkörper gefüllt. Der Cobaltgehalt der Tabletten lag bei 47 Gew.-%, der Mangangehalt bei 15 Gew.-%, der Bariumgehalt bei 1,0 Gew.-%, der Vanadiumgehalt bei 0,8 Gew.-% und der Wolframgehalt bei 0,6 Gew.-%. Die Tabletten hatten bei einer Zylinderhöhe von 5 mm und einem Durchmesser von 5 mm eine Druckfestigkeit von 545 N/cm² auf die plane Zylinderoberfläche und von 110 N auf die gewölbte Formkörperoberfläche sowie eine innere Oberfläche von 117 m²/g.

Nach der Aktivierung dieser verpreßten Metall(hydr)oxid-Pulvermischung wie in Beispiel 1 wurde der Wasserstoffdruck auf 300 bar erhöht.

Anschließend wurden stündlich 560 g Thymol (Reinheit: 99,9 Gew.-%) gemeinsam mit 10 Nm³ Wasserstoff unter einem Druck von 300 bar kontinuierlich von oben nach unten durch das Hochdruckrohr gepumpt, wobei das Thymol vor Eintritt in das Hochdruckrohr auf eine Temperatur von 175°C erhitzt wurde.

Der Thymol-Durchsatz entsprach einer Katalysatorbelastung von 0,4 kg/l Katalysator x h. Der Katalysator war auch nach einer Laufzeit von 6000 h noch hochwirksam.

Im Hydrierprodukt wurde kein Thymol gefunden.

### Beispiel 3

Ein Hochdruckrohr wie in Beispiel 1 wurde unter Inertgas mit 1,4 l durch Tablettierung von Pulvern von Cobalt-, Mangan-, Barium- und Molybdän(hydr)oxiden hergestellter Formkörper gefüllt. Der Cobaltgehalt der Tabletten lag bei 60 Gew.-%, der Mangangehalt bei 18 Gew.-%, der Bariumgehalt bei 1,5 Gew.-% und der Molybdängehalt bei 1,0 Gew.-%. Die Tabletten hatten bei einer Zylinderhöhe von 5 mm und einem Durchmesser von 5 mm eine Druckfestigkeit von 735 N/cm² auf die plane Zylinderoberfläche und von 105 N auf die gewölbte Zylinderoberfläche sowie eine innere Oberfläche von 148 m²/g.

Nach der Aktivierung dieser verpreßten Metall(hydr)oxid-Pulvermischung wie in Beispiel 1 wurde der Wasserstoffdruck bei 200 bar belassen.

Anschließend wurden stündlich 840 g eines Menthol-Isomerengemisches aus 75 Gew.-% d,l-Neomenthol, 22 Gew.-% d,l-Isomenthol und 3 Gew.-% d,l-Neoisomenthol gemeinsam mit 15 Nm³ Wasserstoff unter einem Druck von 200 bar kontinuierlich von oben nach unten durch das Hochdruckrohr gepumpt, wobei das Isomerengemisch und der Wasserstoff vor Eintritt in das Hochdruckrohr auf 165°C erhitzt wurden.

Der Isomerengemisch-Durchsatz entsprach einer Katalysatorbelastung von 0,6 kg/l Katalysator x h. Der Katalysator war auch nach einer Laufzeit von 7600 h noch hochwirksam.

### Beispiel 4

Ein Hochdruckrohr wie in Beispiel 1 wurde unter Inertgas mit 1,4 l durch Tablettierung von Pulvern von Cobalt-, Mangan-, Strontium- und Chrom(hydr)oxiden hergestellter Formkörper gefüllt. Der Cobaltgehalt der Tabletten lag bei 53 Gew.-%, der Mangangehalt bei 16 Gew.-%, der Strontiumgehalt bei 0,9 Gew.-% und der Chromgehalt bei 1,4 Gew.-%. Die Tabletten hatten bei einer Zylinderhöhe von 5 mm und einem Durchmesser von 5 mm eine Druckfestigkeit von 594 N/cm² auf die plane Zylinderoberfläche und von 125 N auf die gewölbte Zylinderoberfläche sowie eine innere Oberfläche von 154 m²/g.

Nach der Aktivierung des Katalysators wie im Beispiel 1 wurde der Wasserstoffdruck auf 250 bar erhöht.

Anschließend wurden stündlich 840 g eines Thymol/Mentholisomeren-Gemisches aus 60 Gew.-% Thymol, 25 Gew.-% Neomenthol und 15 Gew.-% Isomenthol, gemeinsam mit 15 Nm³ Wasserstoff unter einem Druck von 250 bar kontinuierlich von oben nach unten durch das Hochdruckrohr gepumpt, wobei das Reaktionsgemisch vor Eintritt in das Hochdruckrohr auf eine Temperatur von 180°C erhitzt wurde.

Das das Reaktionsrohr verlassende Produkt wurde auf eine Temperatur <60°C abgekühlt und in einem Gasabscheider vom überschüssigen Wasserstoff, der wieder in das Reaktionssystem zurückgeführt wurde, getrennt.

Der Durchsatz des Reaktionsgemisches entsprach einer Katalysatorbelastung von 0,6 kg/l Katalysator x h. Der Katalysator war auch nach einer Laufzeit von 1900 h noch hochwirksam.

Im Hydrierprodukt wurde kein Thymol gefunden.

### Beispiel 5

Ein Hochdruckrohr wie in Beispiel 1 wurde unter Inertgas mit 1,4 l durch Tablettierung von Pulvern von Cobalt-, Mangan-, Barium- und Molybdän(hydr)oxiden hergestellter Formkörper gefüllt. Der Cobaltgehalt der Tabletten lag bei 53 Gew.-%, der Mangangehalt bei 14 Gew.-%, der Bariumgehalt lag bei 1,5 Gew.-% und der Molybdängehalt bei 1,1 Gew.-%. Die Tabletten hatten bei einer Zylinderhöhe von 5 mm und einem Durchmesser von 5 mm eine Druckfestigkeit von 760 N/cm² auf die plane Zylinderoberfläche und von 125 N auf die gewölbte Zylinderoberfläche sowie eine innere Oberfläche von 149 m²/g.

Nach der Aktivierung des Katalysators wie in Beispiel 1 wurde der Wasserstoffdruck auf 300 bar erhöht.

Anschließend wurden stündlich 700 g eines Thymol/Mentholisomeren-Gemisches unter einem Druck von 300 bar kontinuierlich von oben nach unten durch das Hochdruckrohr gepumpt, wobei das Ausgangsprodukt vor Eintritt in das Hochdruckrohr auf eine Temperatur von 175°C erhitzt wurde.

Das Thymol/Mentholisomeren-Gemisch bestand zu 60 Gew.-% aus Thymol und zu 40 Gew.-% aus einem Menthol-Isomerengemisch, wie es bei der Reindestillation des gemäß Beispiel 1 gewonnenen d,l-Menthols durch Vereinigung der Vor- und Nachsieder dieser Destillation anfällt und in der weiteren Folge durch destillative Aufarbeitung des in Beispiel 5 anfallenden Reaktionsproduktes gewonnen wurde. Der Reaktionsgemisch-Durchsatz entsprach einer Katalysatorbelastung von 0,5 kg/l Katalysator x h. Der Katalysator war auch nach einer Laufzeit von 3000 h noch hochwirksam.

Im Hydrierprodukt wurde kein Thymol gefunden.

Nach der destillativen Entfernung der Vor- und Nachsieder wurde das erzeugte d,l-Menthol in einer Reinheit von 99,9 Gew.-% erhalten.

In den wieder in den Reaktionsprozeß zurückgegebenen Destillations-Vor- und Nachläufen reicherten sich keine störenden Nebenkomponenten an, so daß keine Vor- und Nachlaufanteile verworfen werden mußten.

## Patentansprüche

1. Kontinuierliches Verfahren zur Herstellung von d,l-Menthol durch katalytische Hydrierung von Verbindungen, die das Kohlenstoffgerüst des Menthans mit wenigstens einer C=C-Doppelbindung besitzen und in 3-Stellung durch Sauerstoff substituiert sind, mit Wasserstoff und/oder durch katalytische Umlagerung von Stereoisomeren des Menthols in Gegenwart von Wasserstoff, dadurch gekennzeichnet, daß als Katalysatoren dienende trägerfreie Formkörper verwendet werden, die durch Reduktion von Formkörpern aus verpreßten Pulvern von Cobalt-, Mangan- und Erdalkalimetall(hydr)oxiden und gegebenenfalls einem oder mehreren (Hydr)Oxiden von Metallen der V. und/oder VI. Nebengruppe des Periodensystems der Elemente erhältlich sind.

2. Verfahren nach Anspruch 1, wonach die für die Reduktion zu verwendenden Formkörper aus verpreßten Metall(hydr)oxidpulvern (jeweils berechnet als Metall), 40 bis 65 Gew.-% Cobalt, 10 bis 20 Gew.-% Mangan, 0,2 bis 5 Gew.-% Erdalkalimetall und 0 bis 7 Gew.-% Metall der V. und/oder VI. Nebengruppe des Periodensystems der Elemente enthalten, wobei sich die Prozentangaben auf die Gesamtmenge Metall(hydr)oxid-Pulvergemisch beziehen und der Rest zu 100 Gew.-% Sauerstoff ist.

3. Verfahren nach Anspruch 1, dadurch gekennzeichnet, daß ein oder mehrere (Hydr)Oxide von Metallen der V. und/oder VI. Gruppe des Periodensystems der Elemente (Mendelejew), in verpreßtem (Hydr)Oxidpulver vorliegen, deren Gesamtmenge berechnet als Metall, 0,5 bis 7 Gew.-% des gesamten (Hydr)Oxidpulvers beträgt.

4. Verfahren nach Anspruch 1, wonach die Formkörper aus verpreßtem Metall(hydr)oxidpulver eine Druckfestigkeit von 300 bis 800 N/cm² auf die plane Formkörperoberfläche und von 50 bis 200 N auf die gewölbte Formkörperoberfläche (gemessen nach DIN 50 106) besitzen.

5. Verfahren nach Anspruch 1, wonach die Formkörper aus verpreßtem Metall(hydr)oxidpulver eine innere Oberfläche von 30 bis 200 m²/g aufweisen.

6. Verfahren nach Anspruch 1, wonach der Wasserstoffdruck 25 bis 350 bar beträgt.

7. Verfahren nach Anspruch 1, wonach die Umlagerungstemperatur 150 bis 230°C beträgt.

8. Verfahren nach Anspruch 1, dadurch gekennzeichnet, daß während des Verfahrens pro Mol Ausgangsmaterial mindestens die 10-fache molare Menge Wasserstoff den Reaktor passiert.

9. Verfahren nach Anspruch 1, dadurch gekennzeichnet, daß aus dem Reaktionsprodukt der Hydrierung von Thymol bzw. der Umlagerung der Stereoisomeren des Menthols das d,l-Menthol destillativ entnommen wird und die restlichen Reaktionsprodukte unter Zusatz von 10 bis 80 Gew.-% Thymol, bezogen auf die restlichen Reaktionsprodukte, in die Reaktion zurückgeführt werden.

## Claims

1. Continuous process for the preparation of d,l-menthol by catalytic hydrogenation of compounds which have the carbon skeleton of menthane containing at least one C=C double bond and are 3-substituted by oxygen using hydrogen and/or by catalytic rearrangement of menthol stereoisomers in the presence of hydrogen, characterized in that support-free shaped bodies serving as catalysts are used, which are obtainable by reduction of shaped bodies made of pressed powders of cobalt oxide, manganese oxide and alkaline earth metal (hydr)oxides with or without one or more (hydr)oxides of metals of subgroup V and/or VI of the Periodic Table of the Elements.

2. Process according to Claim 1, according to which the shaped bodies made of pressed metal (hydr)oxide powders to be used for the reduction contain (each calculated as metal) 40 to 65 % by weight of cobalt, 10 to 20 % by weight of manganese, 0.2 to 5 % by weight of alkaline earth metal and 0 to 7 % by weight of metal of subgroup V and/or VI of the Periodic Table of the Elements, the percentages being based on the total amount of metal (hydr)oxide powder mixture and the remainder to 100 % by weight being oxygen.

3. Process according to Claim 1, characterized in that one or more (hydr)oxides of metals of group V and/or VI of the Periodic Table of the Elements (Mendeleev) are present in pressed (hydr)oxide powder, whose total amount, calculated as metal, is 0.5 to 7 % by weight of the total (hydr)oxide powder.

4. Process according to Claim 1, according to which the shaped bodies made of pressed metal (hydr)oxide powder have a compressive strength of 300 to 800 N/cm² on the planar shaped body surface and 50 to 200 N on the curved shaped body surface (measured in accordance with DIN 50 106).

5. Process according to Claim 1, according to which the shaped bodies made of pressed metal (hydr)oxide powder have an internal surface area of 30 to 200 m²/g.

6. Process according to Claim 1, according to which the hydrogen pressure is 25 to 350 bar.

7. Process according to Claim 1, according to which the rearrangement temperature is 150 to 230°C.

8. Process according to Claim 1, characterized in that, during the process, at least 10 times the molar amount of hydrogen per mole of starting material passes through the reactor.

9. Process according to Claim 1, characterized in that the d,l-menthol is removed by distillation from the reaction product of the hydrogenation of thymol or the rearrangement of the stereoisomers of menthol and the remaining reaction products, with addition of 10 to 80 % by weight of thymol, based on the remaining reaction products, are returned to the reaction.

## Revendications

1. Procédé continu pour préparer le d,l-menthol par hydrogénation catalytique de composés ayant le squelette carboné du menthane avec au moins une double liaison C=C et sont substitués par l'oxygène en position 3 et/ou par transposition catalytique de stéréoisomères du menthol en présence d'hydrogène, caractérisé en ce que l'on utilise en tant que catalyseurs des corps moulés sans support qui sont obtenus par réduction de corps moulés à partir de poudres comprimées d'(hydr)oxydes du cobalt, du manganèse et des métaux alcalino-terreux et le cas échéant d'un ou plusieurs (hydr)oxydes des métaux des sous-groupes V et/ou VI de la Classification Périodique des éléments.

2. Procédé selon la revendication 1, caractérisé en ce que les corps moulés de poudres comprimées d'(hydr)oxydes métalliques mis en oeuvre pour la réduction contiennent (exprimé en métal dans tous les cas) de 40 à 65 % en poids de cobalt, de 10 à 20 % en poids de manganèse, de 0,2 à 5 % en poids de métal alcalino-terreux et de 0 à 16 % en poids d'un métal des sous-groupes V et/ou VI de la Classification Périodique des éléments, les pourcentages indiqués se rapportant à la quantité totale du mélange de poudres d'(hydr)oxydes métalliques, le solde à 100 % en poids consistant en oxygène.

3. Procédé selon la revendication 1, caractérisé en ce que, dans les poudres comprimées d'(hydr)oxydes, il y a un ou plusieurs (hydr)oxydes de métaux des groupes V et/ou VI de la Classification Périodique des éléments (Mendelejew), avec une teneur totale, exprimée en métal, représentant de 0,5 à 7 % du poids total des poudres d'(hydr)oxydes.

4. Procédé selon la revendication 1, dans lequel les objets moulés à partir de poudres comprimées d'(hydr)oxydes métalliques ont une résistance à la compression de 300 à 800 N/cm² sur la surface plane de ces corps et de 50 à 200 N sur la surface courbe de ces corps (mesure selon la nonne allemande DIN 50 106).

5. Procédé selon la revendication 1, selon lequel les corps moulés à partir de poudres comprimées d'(hydr)oxydes métalliques ont une surface interne de 30 à 200 m²/g.

6. Procédé selon la revendication 1, selon lequel la pression d'hydrogène est de 25 à 350 bar.

7. Procédé selon la revendication 1, selon lequel la température de transposition est de 150 à 230°C.

8. Procédé selon la revendication 1, caractérisé en ce que, dans le cours des opérations, on fait passer dans le réacteur une quantité d'hydrogène représentant au moins 10 mol par mole du produit de départ.

9. Procédé selon la revendication 1, caractérisé en ce que, à partir du produit de l'hydrogénation du thymol ou de la transposition des stéréoisomères du menthol, on sépare le d,l-menthol par distillation et on recycle les autres produits de réaction dans les opérations après adjonction de 10 à 80 % en poids de thymol, par rapport à ces autres produits de réaction.
